# EUROPEAN PATENT APPLICATION

(11) **EP 1 973 206 A1**
(43) Date of publication of application: **24.09.2008**
(21) Application number: 08102320.2
(22) Date of filing: 05.03.2008
(51) Int. Cl.: H01S 3/02, H01S 3/225

(54) **Gas-purged laser system and method thereof**

(30) Priority: 06.03.2007 US 893220 P
(71) Applicant: Alcon RefractiveHorizons, Inc., Fort Worth, Texas 76134 (US)
(72) Inventor: Pettit, George H., Maitland, FL 32751 (US); Downes, George Richard, III, Oviedo, FL 32765 (US); Bott, Steven E., Oviedo, FL 92765 (US)
(74) Representative: Hanna, Peter William Derek

(57) **Abstract**

A gas-purged laser system (100) and method of gas-purging a laser system are disclosed. One embodiment of the laser system comprises an excimer refractive surgical laser system having a laser beam optical path configured to allow purging of a portion of a volume (140) enclosing the laser beam optical path with a gas, and a gas generator (150), operable to generate the purging gas and provide the gas to the volume portion. The portion of the volume can be the entire volume enclosing the laser beam optical path or a selected portion thereof. The gas can be nitrogen gas and the gas generator can be a self-contained nitrogen generator as will be known to those having skill in the art. Embodiments can further comprise a controller (300) for controlling the flow of purging gas in response to received signals representative of various parameters, such as temperature, oxygen level, pressure, humidity and flow rate.

## Description

### CROSS-REFERENCE TO RELATED APPLICATIONS

This application claims priority under 35 U.S.C. §119 to U.S. Provisional Patent Application No. 60/893,220, filed March 6, 2007, the entire contents of which are incorporated herein by reference.

### TECHNICAL FIELD OF THE INVENTION

The present invention relates to systems and methods for purging air from the path of a light beam. More particularly, embodiments of the present invention relate to nitrogen purging of an optical path. Even more particularly, embodiments of the present invention relate to laser vision correction systems having self-contained nitrogen purging systems and methods for purging of a laser beam optical path.

### BACKGROUND

Precise control of laser output power and maintaining the integrity of the laser optical path are critical to achieving successful outcomes in ophthalmic laser surgical procedures. In particular, it is well known that the radiation from an excimer laser, such as is typically used in refractive laser surgical systems, is highly absorbed by oxygen and water vapor when passing through ambient air. As a result of this absorption, the laser beam power output is reduced and ozone gas is produced. Ozone gas is a corrosive gas that can lead to damage of the excimer laser optical elements and optical coatings. The reduction in laser output power and damage to optical path elements that can result from the absorption of an excimer laser beam in ambient air can both lead to inaccuracies in the delivered laser beam power and position that must be accounted for in order to ensure a successful surgical outcome.

One way to reduce such laser beam energy losses and also reduce the formation of harmful ozone is to purge the laser beam path with an inert gas such as nitrogen to displace the ambient air. Prior art systems exist for purging portions of a laser beam path with high purity, dry nitrogen. However, these prior art systems typically supply the nitrogen from evaporation of liquid nitrogen or via compressed gas cylinders. Ultra-cold liquid nitrogen, however, is hazardous and costly to store and handle. Further, it is costly and complex to distribute the nitrogen gas produced from liquid nitrogen to an intended system, such as an excimer laser workstation in a surgeon's office or clinic. Bottled nitrogen gas is more practical than liquid nitrogen, but requires storage space for, and the frequent changing out of, high-pressure gas cylinders. In addition to the time and manpower involved in changing out the gas cylinders, the hazards of high pressure cylinders include handling of the heavy cylinders, connecting and disconnecting of high pressure regulators and the risk of bottle failure leading to a high pressure gas release.

Therefore, there is a need for a self-contained gas purging system and method for purging a laser beam optical path that can reduce or eliminate the problems of prior art gas purging systems. The gas can be, for example, nitrogen gas.

### SUMMARY OF THE INVENTION

Embodiments of the present invention provide a self-contained gas purging system and method for purging a laser beam optical path of a laser system, such as an excimer refractive laser system. The purging gas can be, in a preferred embodiment, nitrogen gas, but can also be another gas suitable for purging of optical systems. Embodiments of the self-contained nitrogen purging system of this invention can comprise a nitrogen generator incorporated as an integral part of a laser system, such as an excimer laser vision correction system or other laser beam delivery system. In one embodiment, ambient air can be filtered to separate out oxygen and other components and generate a nearly pure supply of nitrogen to be supplied as a purge gas along portions of an optical path. In this way, laser beam power output losses can be reduced and the generation of ozone due to laser beam interaction (e.g., an excimer laser beam) with ambient air can be reduced or eliminated.

One embodiment of the present invention comprises a laser system, such as an excimer refractive surgical laser system, having a laser beam optical path configured to allow purging of a portion of a volume enclosing the laser beam optical path with a gas, and a gas generator, operable to generate the purging gas and provide the gas to the volume portion. The portion of the volume can be the entire volume enclosing the laser beam optical path or a selected portion thereof. The gas can be nitrogen gas and the gas generator can be a self-contained nitrogen generator as will be known to those having skill in the art. Embodiments can further comprise a controller for controlling the flow of purging gas in response to received signals representative of various parameters, such as temperature, oxygen level, pressure, humidity and flow rate. The controller can be incorporated within a laser system controller or be a component of the gas generator.

Other embodiments of the present invention can include a method for gas purging of a laser system optical beam path in accordance with the teachings of this invention. Further, embodiments of this invention can be incorporated within any laser system in which it is desirable to purge a portion of the laser beam optical path. Such laser systems can include ophthalmic surgical systems such as refractive excimer laser systems for refractive vision correction. Although the present invention is described herein with reference to an excimer refractive surgical laser system, it is contemplated that the teachings of this invention are equally applicable to, and can be implemented in, any laser system or device in which gas purging of an optical path is desirable.

### BRIEF DESCRIPTION OF THE FIGURES

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a laser system having a self-contained gas generator in accordance with the present invention;
FIGURE 2 is a diagrammatic representation of one embodiment of a refractive surgical laser beam optical path that can be purged in accordance with the present invention; and
FIGURE 3 is a schematic block diagram of an embodiment of a gas generator and purging path of one embodiment of the present invention.

### DETAILED DESCRIPTION

Preferred embodiments of the invention are illustrated in the FIGURES, like numerals being used to refer to like and corresponding parts of the various drawings.

Embodiments of the present invention provide a self-contained gas purging system and method for purging a laser beam optical path of a laser system, such as an excimer refractive laser system. The purging gas can be, in a preferred embodiment, nitrogen gas, but can also be another gas suitable for purging of optical systems. Embodiments of the self-contained nitrogen purging system of this invention can comprise a nitrogen generator incorporated as an integral part of a laser system, such as an excimer laser vision correction system or other laser beam delivery system. In one embodiment, ambient air can be filtered to separate out oxygen and other components and generate a nearly pure supply of nitrogen to be supplied as a purge gas along portions of an optical path. In this way, laser beam power output losses can be reduced and the generation of ozone due to laser beam interaction (e.g., an excimer laser beam) with ambient air can be reduced or eliminated.

Embodiments of the present invention allow a laser system, such as an excimer laser vision correction system, to be self-sufficient as regards generation of nitrogen to protect optical path components and to reduce laser energy losses which would otherwise occur in an unprotected path. Ambient air will typically cause about a 10% loss in laser beam energy for every meter of optical path length. By using the nitrogen gas purge provided by the embodiments of the present invention, this laser beam energy loss can be nearly completely eliminated. Further, embodiments of the present invention eliminate the need for external nitrogen gas cylinders which must be routinely exchanged as the nitrogen gas is used. Laser system embodiments of the present invention can thus be more user friendly and require less maintenance, less storage space and be less costly as a user will not be required to store or exchange gas cylinders for purging of the system.

One example of a nitrogen gas generator that can be incorporated into embodiments of the present invention is a membrane-separation type nitrogen generator. This type of generator is capable of producing dry nitrogen of up to 99.9% purity. Such a system will typically require a source of compressed air, which can be either an integral compressor or a separate air compression unit. A nitrogen generator of this type can provide for control of gas flow rate and purity of nitrogen production while delivering a sufficient flow rate to purge the optical path of a laser system through maximum intended use. Further, nitrogen generation systems of this type can be relatively maintenance free, requiring, for example, an annual filter/membrane exchange as the only routine maintenance of note.

A nitrogen membrane type nitrogen generator can provide a low-cost, highly efficient means of separating air into its component gases. Because this technology requires no moving parts and consumes relatively little energy, it is economical to operate and maintain-the main expense is the energy required to provide a stream of compressed feed air. Such a system typically will comprise gas pressure control valves and instruments, a coalescing filter and carbon filter (which removes particles and liquid vapors from the feed line), and the nitrogen membrane module.

The nitrogen membrane module may consist of bundles of hollow fiber, semipermeable membranes. Each fiber has a circular cross-section and a uniform core through its center. The wall thickness of each fiber is thus consistent, which contributes to the physical strength of each membrane. Because the fibers are so small (about the diameter of a human hair), a great many can be packed into a limited space, providing an extremely large membrane surface area that can produce a relatively high volume product stream.

The hollow fibers are assembled parallel to a central core tube, and the bundle is inserted into an outer case to form the air separation module. Compressed air is introduced into the center of the fibers at one end of the module and contacts the membrane as it flows down to fiber bores. Oxygen, water vapor and other "fast gases" pass through the outside of the fibers. The oxygen-rich gas stream then flows through the fiber bundle to the periphery of the case, where it is discharged as a by-product.

While all but a small fraction of the oxygen passes through the membrane material to the exterior of the hollow fibers, most of the nitrogen present in the feed air is contained within the hollow fiber membrane. Since water vapor passes through the membrane along with the oxygen, this nitrogen product is essentially moisture-free. The nitrogen stream emerges at a pressure slightly below that of the feed air pressure.

Because the membrane module contains no moving parts, it requires no maintenance. The only attention such a nitrogen generation system typically needs is an occasional recalibration of the oxygen analyzer and a filter change. While the embodiments of the present invention are described with reference to a membrane type nitrogen generator such as the above, any suitable nitrogen generator is contemplated to be within the scope of the present invention.

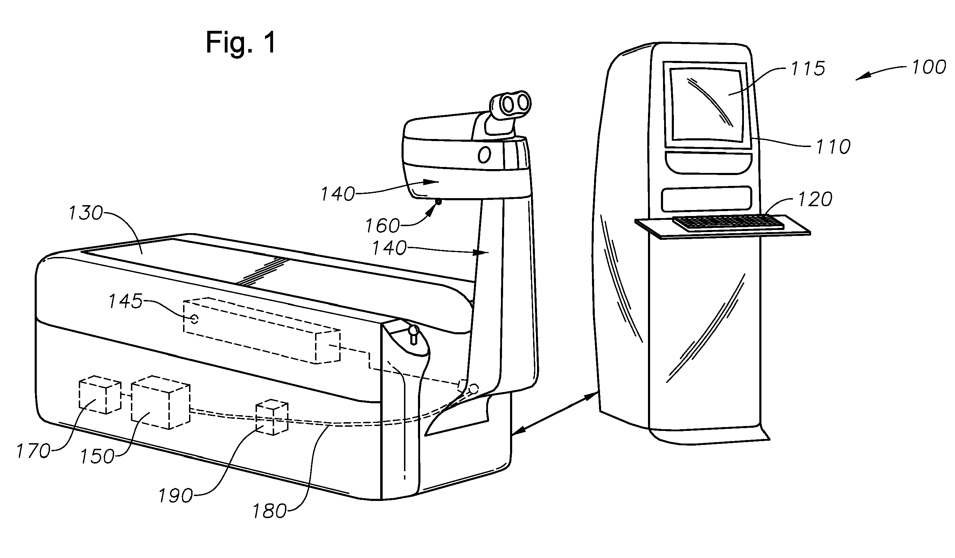

FIGURE 1 is a diagrammatic representation of one embodiment of a laser system 100 comprising a self-contained gas generator 150. Laser system 100 can include a monitor 110 that has touch screen 115. Touch screen 115 can provide a GUI that allows a user to interact with laser system 100. A user may also interact with laser system 100 via a keyboard 120 and/or mouse. Laser system 100 further includes a patient support structure 130 and a laser optical path enclosure 140. Laser optical path enclosure 140 houses and encloses at least a portion of the optical path and corresponding optical elements that are used to guide a laser beam from a laser source 145 to an output port 160 of laser optical path enclosure 140 and then onto a surgical site of a patient's eye. The optical elements and details of the optical path are not shown as they can vary and such optical path designs will be known to those having skill in the art. Laser system 100 is provided by way of example and embodiments of the present invention can comprise any suitable laser system in which it is desirable to purge an optical path. Preferred exemplary systems include laser refractive vision correction systems, such as shown in FIGURE 1.

Gas generator 150 can comprise a separate or integral compressor 170 to supply compressed air for the gas generation process. The air supplied by compressor 170 is preferably oil-free. In a preferred embodiment, gas generator 150 is a nitrogen gas generator, such as a membrane-type nitrogen generator as will be known to those having skill in the art. Nitrogen gas generator 150 is operable to produce and supply purified nitrogen gas to purge at least a portion, and in some cases all, of an enclosed volume within laser optical path enclosure 140 enclosing a laser beam optical path from laser source 145 to laser output port 160. A portion of a nitrogen purge gas supply system is represented by gas supply path 180, which can comprise valves and piping and control systems for regulating the flow (supply) of nitrogen gas from gas generator 150 to laser optical path enclosure 140 for purging of the laser beam optical path.

Self-contained gas generator 150 permits the generation and supply of, for example, nitrogen gas on a need basis. Storage of highly compressed gas bottles or liquefied nitrogen, as in the prior art, is therefore obviated. Embodiments of the present invention can also include a reservoir 190 for storing enough gas to ensure that a failure of gas generator 150 will not preclude completing a surgical procedure that may be in progress. Further, reservoir 190 will allow for shutting down of gas generator 150 during the firing of the laser source 145 and delivery of the laser beam to a patient's eye. Because the nitrogen generator 150 pump and compressor 170 can be sources of noise and vibration, the ability to shut both down during an excimer laser treatment (e.g., for about 60 seconds) while supplying needed nitrogen purge gas from reservoir 190 to the optical path is an advantage of the present invention that will contribute to good surgical outcomes.

The embodiments of the present invention provide further advantages of on demand purge gas generation, low maintenance and service costs, decreased space and storage requirements (no high pressure cylinders required), and self-containment within the body of the laser system 100 (e.g., an excimer laser refractive surgery workstation). The main replacement/maintenance cost expected to be incurred as a result of the gas generation aspects of the present invention are the replacement of the membrane (molecular filter) used to separate a chosen gas (e.g., nitrogen) from the other components of ambient air.

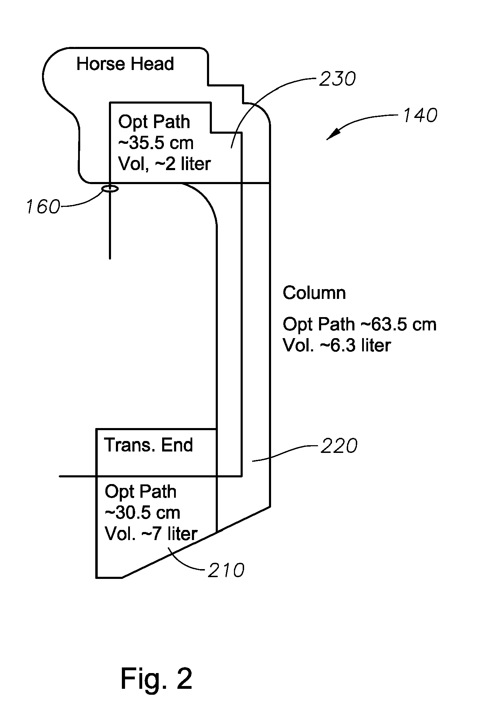

Laser optical path enclosure 140 can be configured into one or more purging zones which can be contained using a thin optical window at each end of the contained volume so as not to interfere with the laser beam as it traverses the optical path. Nitrogen gas can be introduced towards the top of a purge zone and allowed to leak out in a controlled manner or released using a low-pressure relief valve, for example. FIGURE 2 is a diagrammatic representation of one embodiment of a refractive surgical laser beam optical path within a laser optical path enclosure 140 that can be purged in accordance with the present invention.

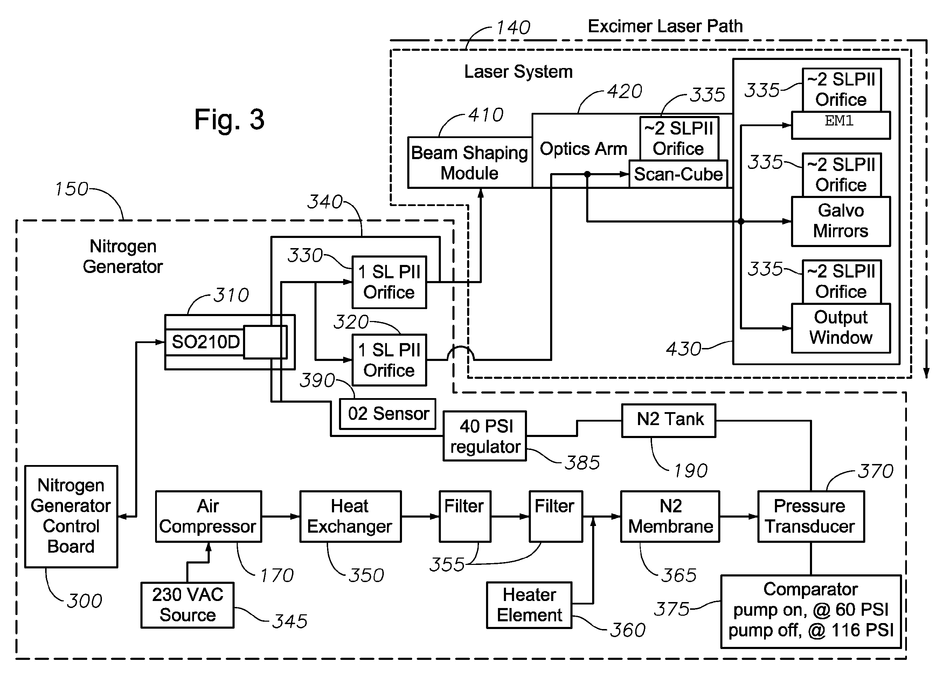

In the embodiment of FIGURE 2, optical path enclosure 140 includes three purging zones 210, 220, and 230. Either or all of these three purging zones can be purged with nitrogen gas supplied from gas generator 150 via gas supply path 180 (as shown more clearly in FIGURE 3). FIGURE 2 illustrates an exemplary path configuration and path lengths with estimated gas volumes.

FIGURE 3 is a schematic block diagram of an exemplary gas generator 150 and purging path of one embodiment of the present invention. In the embodiment of FIGURE 3, gas generator 150 is a nitrogen gas generator. Nitrogen generator 150 includes a control board 300 for controlling the generation, flow and gas characteristics of the generated nitrogen gas. Control board 300 can be a printed circuit board as will be known to those having skill in the art and can be integral to gas generator 150, as shown in FIGURE 3, or can be a part of a laser system 100 control board or control system. Control board 300 provides a control signal to solenoid 310, which controls the flow and distribution of nitrogen gas to the various portions of the optical path within optical path enclosure 140 via orifices 320 and 330. Orifices 320 and 330 can be electromechanically controlled valves or other such variable or constant flow orifices as will be known to those having skill in the art. Solenoid 310 can also provide nitrogen gas directly to the optical path via bypass line 340 in some embodiments.

Control board 310 can take as input signals representative of oxygen level (e.g., from oxygen sensor 390), pressure (flow) from, e.g., pressure transducer 370, temperature, humidity, nitrogen purity, and any other such parameters, including user set parameters, that may be useful in regulating quantity and quality of the nitrogen supplied to the laser system 100 optical path. Based on such input signals, control board 300 can generate and provide control signal(s) to solenoid 310 which can in turn control nitrogen flow by controlling the opening and closing of orifices 320, 330, and 335.

Gas generator 150 can further comprise air compressor 170, power supply 345, heat exchangers 350, filters 355, heater element 360, nitrogen membrane 365, pressure transducer 370, comparator 375, gas reservoir 190 (which can also be external to gas generator 150), and pressure regulator 390. These components, in various combinations, will be recognized by those having skill in the art as being common to nitrogen gas generators of the membrane type and operable to perform functions necessary to the proper operation of gas generator 150. The gas generator 150 of FIGURE 3 is exemplary, and the components shown contemplated to be operable to perform functions as known to those skilled in the art.

Gas generator 150 generates, in this case, nitrogen gas and provides the gas to portions of the optical path within optical path enclosure 140 as discussed above. These portions of the optical path can include, in an exemplary embodiment of FIGURE 3, beam shaping module 410, optics arm 420, and optics head 430. Although the embodiment of FIGURE 3 shows just these three discrete portions of the optical path, the present invention contemplates within its scope various different configurations, having a differing number of discrete portions as may be desired for a particular laser system 100 optical path. Nitrogen gas can be supplied to any combination of some or all of the optical path portions by gas generator 150 as desired and controlled by a user. Nitrogen purging can be automatically initiated, keyed to an event at laser system 100, or can be manually initiated by a user as desired. Although not shown, embodiments of the present invention are contemplated to include the various valves, solenoids, control mechanisms, etc., required to control the flow of a fluid in a system as will be known to those having skill in the art.

Control board 300 can be onboard or connected to gas generator 150 and laser system 100. Control board 300 can include a processor, such as an Intel processor (Intel is a trademark of Intel Corporation of Santa Clara, California), a primary memory (e.g., RAM, ROM, Flash Memory, EEPROM or other computer readable medium known in the art) and, in some embodiments, a secondary memory (e.g., a hard drive, disk drive, optical drive or other computer readable medium known in the art). A memory controller can control access to secondary memory. Control board 300 can include I/O interfaces, such as a touch screen interface, mouse and/or keyboard. A video controller can control interactions over the touch screen interface. Similarly, an I/O controller can control interactions over other I/O interfaces. Control board 300 can include a variety of input devices. Various components of control board 300 can be connected by a bus.

The secondary memory can store a variety of computer instructions that include, for example, an operating system such as a Windows operating system (Windows is a trademark of Redmond, Washington based Microsoft Corporation) and applications that run on the operating system, along with a variety of data. More particularly, secondary memory can store a software program that can control the generation and flow of nitrogen for a surgical procedure. During execution by the processor, portions of the software program can be stored in secondary memory and/or in primary memory.

Although the present invention has been described in detail herein with reference to the illustrated embodiments, it should be understood that the description is by way of example only and is not to be construed in a limiting sense. It is to be further understood, therefore, that numerous changes in the details of the embodiment of this invention and additional embodiments of this invention will be apparent, and may be made by, persons of ordinary skill in the art having reference to this description. It is contemplated that all such changes and additional embodiments are within scope of the invention as claimed below.

## Claims

1. A laser system (100), comprising:
a laser beam optical path configured to allow purging of a portion of a volume (140) enclosing the laser beam optical path with a gas; and
a gas generator (150), operable to generate the purging gas and provide the gas to the volume portion.

2. The laser system of claim 1, wherein the portion of the volume (140) is the volume enclosing the laser beam optical path.

3. The laser system of claim 1, wherein the gas generator (150) is a nitrogen gas generator and wherein the gas is nitrogen gas.

4. The laser system of claim 1, further comprising a controller (300) for controlling the flow of purging gas in response to received signals representative of various parameters.

5. The laser system of claim 4, wherein the various parameters are selected from the group including temperature, oxygen level, pressure, humidity and flow rate.

6. The laser system of claim 1, wherein the laser system (100) is an excimer refractive surgical laser system.
